# EUROPEAN PATENT SPECIFICATION

(11) **EP 0 375 440 B1**
(45) Date of publication and mention of the grant of the patent: **12.04.1995**
(21) Application number: 89313465.0
(22) Date of filing: 21.12.1989
(51) Int. Cl.: A61B 5/0408, D04H 1/46

(54) **An electrode for use on living tissue**
Elektrode zur Verwendung auf lebendem Gewebe
Electrode destinée à l'usage sur tissu vivant

(30) Priority: 23.12.1988 JP 325510/88; 24.01.1989 JP 13180/89
(43) Date of publication of application: 27.06.1990
(73) Proprietor: JAPAN GORE-TEX, INC., Setagaya-ku Tokyo 156 (JP)
(72) Inventor: Mitsui, Kazuyuki c/o Japan Gore-Tex, Inc., Wake-Gun Okayama-Ken 709-02 (JP); Kato, Hiroshi c/o Japan Gore-Tex, Inc., Wake-Gun Okayama-Ken 709-02 (JP); Fukui, Yasuhiro c/o Japan Gore-Tex, Inc., Wake-Gun Okayama-Ken 709-02 (JP); Chikamori, Yoshihiro c/o Japan Gore-Tex, Inc., Wake-Gun Okayama-Ken 709-02 (JP)
(74) Representative: McCallum, William Potter

(56) References cited:
- EP-A- 0 008 460
- EP-A- 0 275 628
- GB-A- 2 045 088
- GB-A- 2 179 555
- US-A- 4 187 390

## Description

The present invention relates to an electrode for use on living tissue, for example in stimulation and the measurement of electric potentials of a human body such as in electrocardiography.

In the case of electrodes conventionally used for the measurement of electric potentials of the human body (e.g. electrocardiographic electrodes) or for stimulation in pacemakers and therapy, etc., the electrodes are retained on the surface of the skin by suction, using a suction disc at each measurement position on the body. Usually, the electrodes are placed in position following a pre-treatment in which the surface of the skin is coated with a conductive jelly.

In another system which is also used, the electrodes are fixed in place by means of adhesive tape, as for example described in GB-A-2 045 088. In such cases, the electrodes used are generally metal electrodes.

With the abovementioned conventional electrodes attached using suction discs, there are difficulties in electrode retention. For example, in cases where the negative pressure of the suction discs is weak, the suction force may be last as a result of slight movements of the body, or perspiration. Accordingly, it is difficult to perform electrocardiographic measurements over a long period of time using such electrodes. Furthermore, as a result of the need to apply a conductive jelly to the surface of the skin in order to improve the conductivity of the suction attachment, the setting of the electrodes on the body takes time, which is especially inconvenient in cases of emergency.

On the other hand if the negative pressure of the suction discs is too great, subcutaneous haemorrhaging may occur. Accordingly, there are unavoidable limitations on the negative pressure that can be used.

In cases where adhesive tape is used, reddening of the skin commonly occurs. This gives the patient an unpleasant sensataion, and the patient commonly experiences discomfort in the case of long-term recording.

Furthermore, with conventional metal electrodes, there is a feeling of weight when the electrodes are applied to the measurement positions on the skin. Also, since such electrodes are rigid and therefore do not readily conform to the contours of the skin surface, the effective contact area is limited, and the electrodes may easily slip off.

According to the invention there is provided an electrode, particularly for use on living tissue, for making electrically conductive contact with a surface and comprising an electrode having a portion for contacting said surface, the contact portion comprising a porous material, characterised in that said porous material contains from 5 to 90% by weight polytetrafluoroethylene and from 10 to 95% by weight conductive powder and in that the pores of the porous material have sufficient size to allow the passage of water vapour therethrough.

The electrode material may be made porous by any suitable pore-forming technique that is capable of creating void spaces within the material. An electrode of such material is soft, flexible, moisture vapour permeable, and is lighter in weight compared to conventional electrode materials. This enables a reliable and stable attachment of the electrode to living tissue to be achieved.

The preferred electrode material is made porous by a fibrillization process that produces a microstructure of nodes interconnected by fibrils, such that void spaces or pores exist between the fibrils, and the nodes interconnected by the fibrils are substantially connected to each other. The conductive powder collects in these nodes portions, so that the concentration of said powder is enriched in the node portions.

Since the nodes are substantially connected to each other, electrical conduction is obtained from the areas of enriched conductive powder concentration formed as described. Accordingly, the material has an appropriate effect as an electrode for measuring electric potentials.

As a result of the polytetrafluoroethylene content of not less than 5 percent by weight (and preferably not less than 20 percent by weight), the abovementioned fibrillized portions of the preferred electrode material can be formed effectively. Furthermore, in regard to the upper limit of 90 percent by weight (and preferably not more than 70 percent by weight) of the polytetrafluoroethylene content, this limit is set in order to ensure a content of at least 10 percent by weight (and preferably at least 30 percent by weight) of conductive powder so as to obtain an electrical conductivity appropriate for an electrode.

The electrode in accordance with the present invention may be made in various shapes, e.g. circular, elliptical, square, rectangular, etc. At least one surface may be partly or completely coated with a suitable adhesive to allow the electrode to be attached stably to the skin without significantly inhibiting the electrical conductivity or water vapour permeability properties of the electrode.

Additionally, the electrode may be formed with a connecting part which projects from one edge in order to facilitate the connection of lead wires, etc.

Any suitable method for forming pores or void spaces within a PTFE/conductive powder material may be used to create the porous electrode material of the present invention so long as pores of a size sufficient to pass moisture vapour are created. For example, a powder with a pore-forming action may be mixed with the conductive powder, and the pores formed by removing this pore-forming agent (by heating, or solvent extraction, etc.) following the moulding process.

Preferably, however, a material formed by mixing a conductive powder with a polytetrafluoroethylene resin (hereafter referred to as "PTFE") is fibrillized by a rolling or drawing treatment, so that large numbers of fibrils and nodes are formed into a microstructure wherein the fibrils interconnect numerous small nodes which are substantially connected to each other, the resulting sheet material being used as the electrode material.

This fibrillized material may be produced by the following steps (1) to (6):
(1) A mixture formed by adding a conductive powder and a liquid lubricant to finely powdered PTFE is kneaded into a paste-like substance.
(2) This paste-like substance is formed into a sheet, tube or rod by compression, extrusion, drawing or rolling, or by a combination of two or more of these techniques. This step initially fibrillizes the material.
(3) The liquid lubricant is then removed from the shaped material by heating or extraction, etc.
(4) If necessary, the material is next subjected to a drawing or rolling treatment in at least one direction, so that the material is further fibrillized. This is preferably done while heating the material to a temperature less than the crystalline melt point of PTFE.
(5) In cases where most of the fine nodes formed as a result of the abovementioned fibrillization are separated from each other, a further compression treatment may be performed so that the nodes are connected to each other to a substantial degree. This step is performed if a reduction in porosity or increase in density or conductivity is desired.
(6) The material obtained as a result of steps (1) to (3), steps (1) to (4) or steps (1) to (5) is sintered at a temperature greater than the crystalline melt point of PTFE.

The abovementioned step (5) may be performed by means of pressing plates or rolls, etc., and, if preferred, step (5) may be performed after step (6).

The abovementioned steps (1) to (6) are generally described in U.S. Patents Nos. 3,953,566; 3,962,153; and 4,187,390. These patents describe porous expanded fibrillized PTFE in which the expansion occurs during step (4) described above. If the material is not subjected to step (4), then it is not considered an expanded material, although it is still considered fibrillized.

The conductive powder used in the electrode material in accordance with the invention is preferably a carbonaceous powder such as carbon black or graphite, etc. However, it would also be possible to use metals such as gold, tantalum or titanium, etc., oxides of such metals, or powdered Raney metals, etc. Such substances may be used alone or in mixtures.

It is desirable that the conductive powder has a mean particle diameter of 10 microns or less. A powder with a mean particle diameter exceeding 10 microns causes difficulties in working in processes such as the abovementioned steps (2) and (3), so that reliable fibrillization and pore size adjustment becomes difficult to achieve.

Furthermore, the amount of conductive powder used in the material is at least 10 percent by weight, preferably at least 30 percent by weight, and more preferably from 40 to 80 percent by weight. In this way, the volume resistivity of the material can be lowered to 1.0 ohm-cm or less.

Examples of liquid lubricants which can be used in forming the paste in the abovementioned step (1) include liquid hydrocarbons such as petroleum, solvent naptha and white oil, etc., and other substances such as ethylene glycol, glycerine, water, polyethylene oxide and phthalic acid esters, etc. The amount of liquid lubricant used is generally from 18 to 220 percent by weight relative to the amount of PTFE used. Kneading and adjustment can be performed using appropriate well known methods. If desired, waxes or other materials which enhance water-repelling properties may be added as supplementary raw materials.

A sheet with, for example, a fibril length of 0.08 to 22 microns, a density of 0.18 to 1.15 g/cm³, and a Gurley number of 0.9 seconds or greater can be reliably obtained as described above.

Furthermore, it has been confirmed by microscopic observation that in the conversion of the precursor material into a porous material by a drawing process such as in the abovementioned step (4), most of the conductive powder mixed with the PTFE collects in the node portions. Powder also adheres to the surfaces of the fibrils. Since the node portions are in contact with each other or are connected to each other, an electrical conductivity appropriate for an electrode can be reliably obtained. In cases where a fine carbon powder with a particle size of 0.1 microns or less (e.g. carbon black, etc.) is used, especially, such a structure can also be obtained by using an excess of liquid lubricant and performing the aforementioned steps (1) to (3) only (i.e without any need to perform the steps from (4) onwards). Furthermore, conductive powder adhering to the surfaces of the fibrils can be brought into close contact, thus reinforcing the electrical conductivity by utilizing an appropriate compression treatment step (5) following the abovementioned drawing treatment step (4), either before or after the heat treatment step (6). In cases where such a compression treatment results in an insufficient gas permeability, perforations through the material can be formed by laser working or discharge working, etc.

A number of examples of electrodes in accordance with the invention will now be described with reference to the accompanying drawings, in which:-
Figure 1 is a plan view showing a pair of electrodes constructed in accordance with one example of the present invention;
Figure 2 is a diagram which illustrates the use of the electrodes of Figure 1 applied to the heart for electrocardiographic measurement;
Figures 3 and 4 are graphs which show the results of electrocardiographic measurements obtained using the electrodes of the first example;
Figures 5 and 6 show corresponding measurement results obtained using conventional copper electrodes;
Figure 7 is a plan view illustrating another example of an electrode in accordance with the present invention;
Figure 8 is a diagram which illustrates the use of electrodes of the type shown in Figure 7 applied to the right arm, left arm and right ankle of a pateint;
Figure 9 is a scrap diagram which illustrates an electrical connection to an electrode in the arrangement of Figure 8 using an alligator clip;
Figure 10 is a diagram which is similar to that of Figure 9 but showing the connection to a comparison metal electrode;
Figure 11 is a graph showing electrocardiographic measurement results obtained using electrodes of Figure 7, and electrocardiographic measurement results obtained using comparative metal electrodes, in an arrangement as shown in Figure 8;
Figure 12 is a plan view illustrating another example of an electrode in accordance with the present invention;
Figure 13 is a diagram showing a disposable electrocardiographic electrode assembly incorporating the electrode shown in Figure 12;
Figure 14 is a graph which shows electrocardiographic measurement results obtained using electrodes of the type shown in Figures 12 and 13 (using physiological saline and a conductive jelly in separate experiments), and electrocardiographic measurement results obtained using comparative metal electrodes with a conductive jelly.

### EXAMPLE 1

A mixture consisting of 40 percent by weight of powdered PTFE and 60 percent by weight conductive carbon black (Ketchen Black EC) was prepared. 70 parts by weight of a liquid lubricant was mixed with 100 parts by weight of the PTFE/carbon black mixture so that a paste was obtained. This paste was temporarily moulded into a cylindrical shape, and was then formed into a sheet with a thickness of 0.12 mm by compression, paste extrusion and rolling. Next, the sheet was fixed so that it would not shrink in the direction of rolling, and was heated in this state so that the liquid lubricant was removed (and so that dimensional stability was ensured). In this way, an electrode material was obtained. The volume resistivity of this material was 0.5 ohm-cm, and the Gurley number (indicating gas permeability) was 80 seconds.

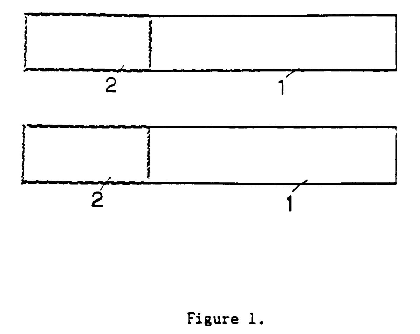

The electrode material so obtained was used to form electrodes (1) with a width of 5 mm and a length of 30 mm as shown in Figure 1, one end portion of each electrode (1) being coated on one side with an adhesive to provide an adhesive part (2) (with a length of 10 mm).

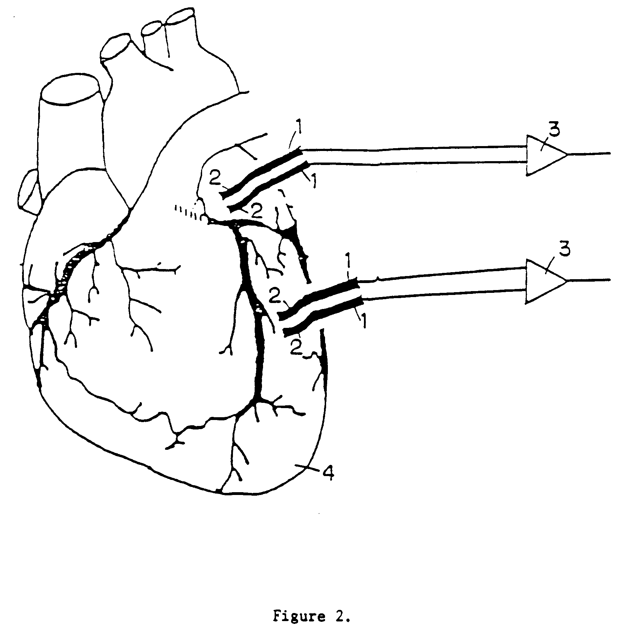

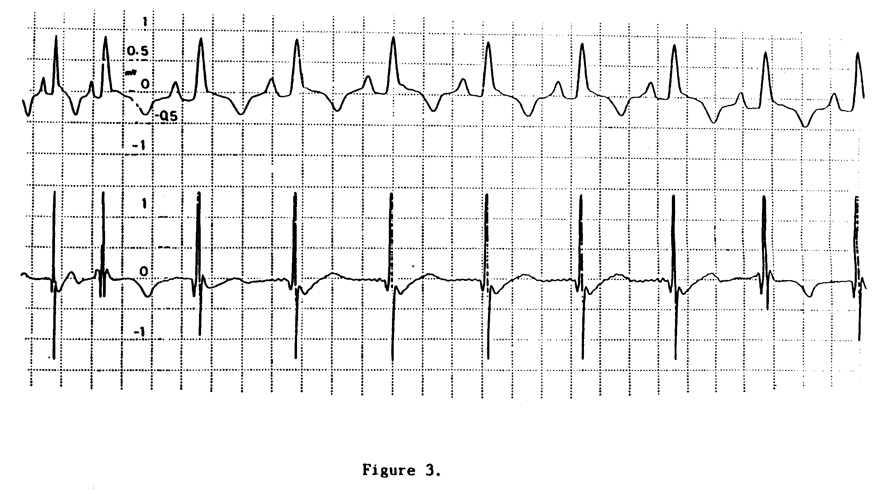

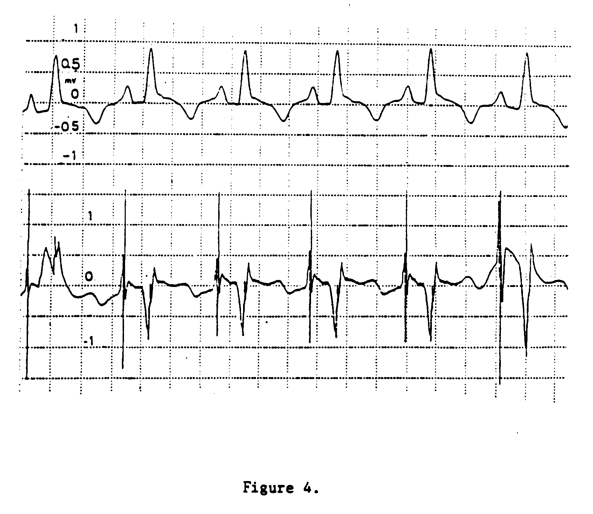

For test purposes the adhesive ends (2) of two pairs of said electrodes (1,1) were applied to the left ventricle and left atrium areas of a heart (4) as shown in Figure 2 so that respective bipolar arrangements were obtained, and the opposite ends of the electrode pairs were connected to differential amplifiers (3). The waveforms from the respective electrodes were input using the differential amplifiers (3), and the left ventricle and left atrium area results obtained are shown respectively in Figures 3 and 4.

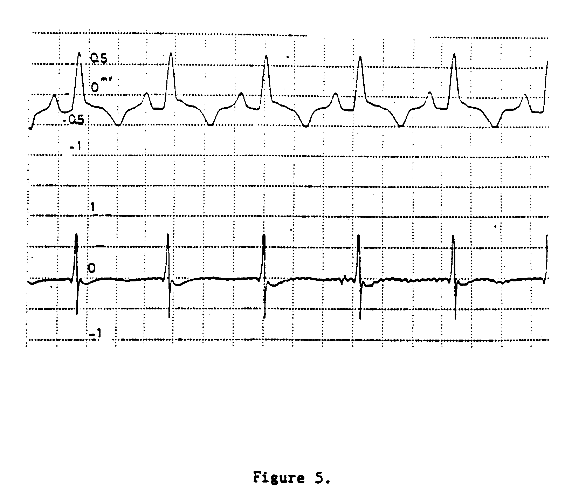

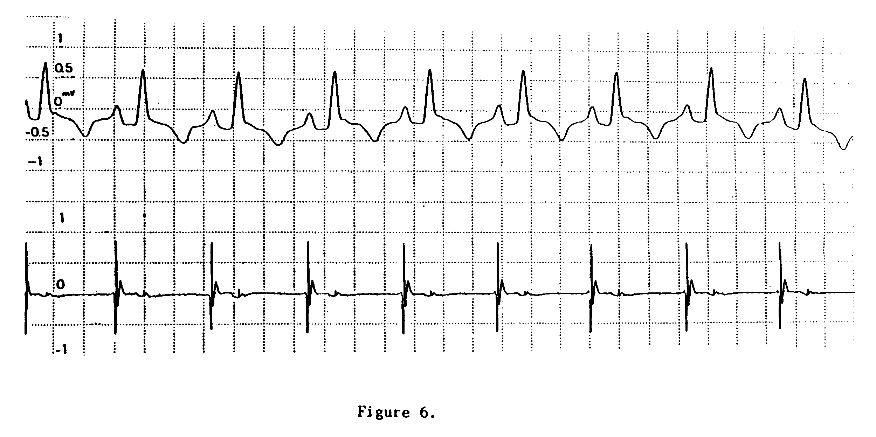

As a comparison for the waveforms obtained using the electrodes in accordance with the invention as described above, activity potential waveforms for the right ventricle and right atrium area were obtained using bipolar electrode arrangements comprising conventional copper electrodes and are shown respectively in Figures 5 and 6.

In Figures 3 to 6, electrocardiographic waveforms obtained from electrodes placed on the right wrist and left foot of a person are shown in the upper portion of each figure, and waveforms obtained from the electrodes placed over the heart area as described above are shown in the lower portion of each figure. It is clear that the electrodes in accordance with the present invention produce measurement results that are equal to or better than the measurement results obtained using conventional copper electrodes.

The electrodes (1) shown in Figure 1 have a simple rectangular shape. However, in a more preferred configuration, the adhesive part (2) of each electrode (1) is formed in the shape of a square whose width is greater than the width of the remainder of the electrode (1).

Furthermore, the electrodes in accordance with the invention have a desirable moisture vapour permeability, so that faulty adhesion due to perspiration, etc., can be avoided.

### EXAMPLE 2

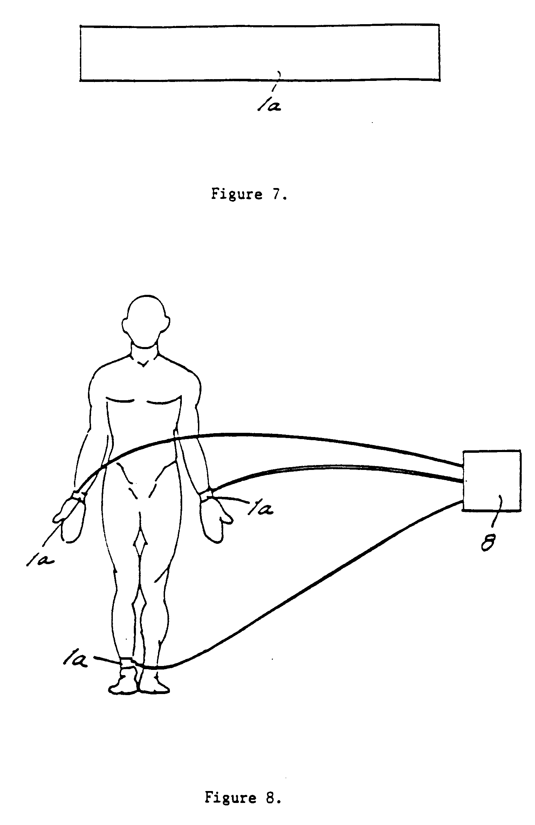

Using electrode material as obtained in Example 1, an electrode (1a) for application to a body by wrapping around the ankle or wrist was manufactured as shown in Figure 7 (width of electrode: 15 mm, length of electrode: 225 mm).

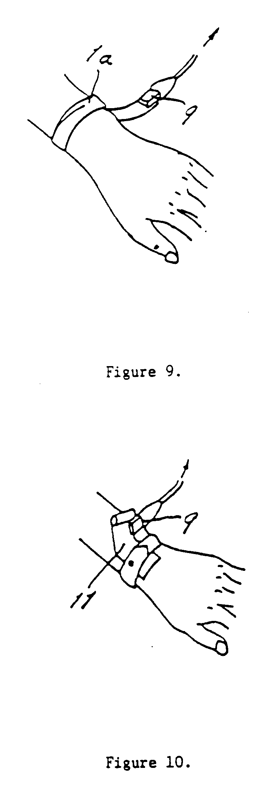

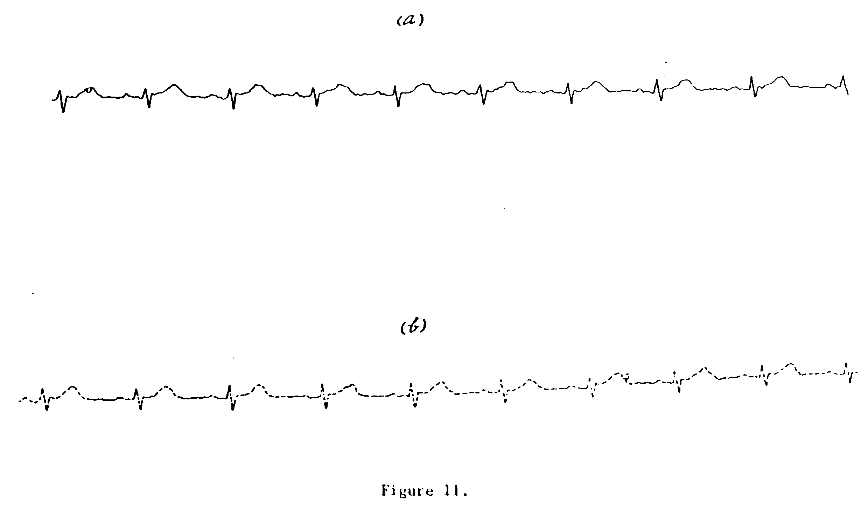

Samples of this electrode (1a) were wrapped around the right and left wrists and the right ankle of a person as shown in Figure 8, and each of the electrodes was connected to an electrocardiograph 8 by means of an alligator clip as shown (for example) in Figure 9. An electrocardiogram was measured using this electrode arrangement. Separately, metal electrodes (11) used for comparison purposes were connected by means of alligator clips (9) as shown in Figure 10, and an electrocardiogram was recorded using this arrangement as well. The results obtained are compared in Figure 11, wherein Figure 11 (a) shows the results using the electrodes in accordance with the invention and 11(b) the results from the comparison electrodes. It will be seen that the invention produced measurement results that were equal to or better than the measurement results produced using the comparison electrodes.

Furthermore, in obtaining these measurements, it was confirmed that the electrodes in accordance with the invention do not require the use of a conductive jelly (which was necessary in the case of the comparison metal electrodes). Instead, it is sufficient if a small amount of physiological saline solution is placed between the electrodes and the body. Such an arrangement eliminates the unpleasant sensation experienced by the patient when a conductive jelly is used. Furthermore, peeling of the electrodes due to perspiration, which is a problem in the case of conventional electrodes, is completely eliminated when using electrodes in accordance with the invention. Indeed, it was confirmed that perspiration actually increases the degree of contact between such electrodes and the skin.

### EXAMPLE 3

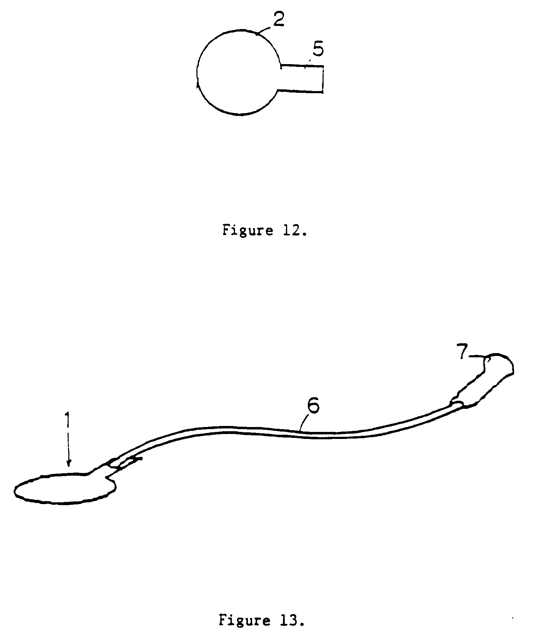

Using an electrode material obtained in the same manner as in Example 1, an electrode (1) was prepared which has a connecting part (5) with a width of 5 mm and a length of 10 mm projecting from one edge of a flat, circular adhesive part (2) having a diameter of 20 mm, as shown in Figure 12. An electrocardiographic electrode assembly was obtained by connecting a cord (6) and a connector (7) (as conventionally used with disposable electrocardiographic electrodes) to the connecting part (5) of the abovementioned electrode (1) as shown in Figure 13.

Electrodes (1) produced in this way were attached to the skin of the right hand and left foot (ground) of a patient, and an electrocardiogram was taken. In this case, the attachment of the electrodes was effected in conjunction with physiological saline in one experiment and a conductive jelly in a second experiment. Furthermore, electrocardiographic electrodes made of metal were separately used in a comparative example, these electrodes being attached using a conductive jelly, and similar measurements were performed.

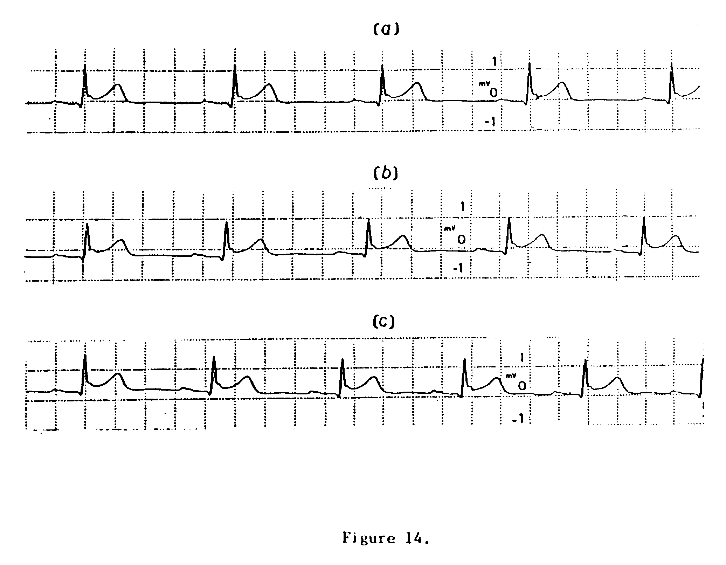

The measurement results obtained are shown in Figure 14, in which (a) shows the results obtained with the electrodes in accordance with the invention attached using physiological saline, (b) shows the results obtained with the electrodes in accordance with the invention attached using a conductive jelly, and (c) shows the results obtained with the comparison metal electrodes attached using a conductive jelly. It can be seen that there is virtually no difference between these measurement results (a), (b) and (c), demonstrating that effective measurement results can be obtained by using electrodes in accordance with the present invention in conjunction with physiological saline.

Furthermore, in regard to the shape of the adhesive part (2) of the electrode shown in Figure 12, it is not absolutely necessary that this part be perfectly circular in shape, and in some cases the part may be elliptical or oval or some other shape. Moreover, the connecting part (5) may be of any desired shape, as long as it prevents the adhesive part (2) from being affected by the connected cord (6).

## Claims

1. An electrode, particularly for use on living tissue, for making electrically conductive contact with a surface, said electrode having a portion for contacting said surface, the contact portion comprising a porous material, characterised in that said porous material contains from 5 to 90% by weight polytetrafluoroethylene and from 10 to 95% by weight conductive power and in that the pores of the porous material have sufficient size to allow the passage of water vapour therethrough.

2. An electrode according to claim 1, wherein the porous material has a microstructure of nodes interconnected by fibrils, such that void spaces exist between the fibrils, and the nodes interconnected by the fibrils are substantially connected to each other.

3. An electrode according to claim 2, wherein the porous material has been rendered porous by a fibrillization process.

4. An electrode according to any one of claims 1 to 3, wherein the conductive powder is carbon black.

5. An electrode according to any one of claims 1 to 3, wherein the conductive powder is metallic.

6. An electrode according to any one of the preceding claims, wherein the particle size of the conductive powder is not more than 10 microns.

7. An electrode according to any one of the preceding claims, having at least one surface which is at least partly coated with an adhesive.

8. An electrode according to any one of the preceding claims, wherein the electrode is rectangular in shape.

9. An electrode according to any one of claims 1 to 7, wherein the electrode is circular or elliptical in shape.

10. An electrode according to claim 8 or claim 9, having a connecting part projecting from one edge.

## Patentansprüche

1. Eine Elektrode, insbesondere zur Verwendung auf lebendem Gewebe, um einen elektrisch leitenden Kontakt mit einer Oberfläche herzustellen, wobei die genannte Elektrode einen Abschnitt zum Kontaktieren der genannten Oberfläche aufweist, und wobei dieser Kontakt-Abschnitt ein poröses Material aufweist,
**dadurch gekennzeichnet,**
daß das genannte poröse Material von 5 bis 90 Gew.-% Polytetrafluorethylen und von 10 bis 95 Gew.-% leitfähiges Pulver enthält und daß die Poren des porösen Materials eine ausreichende Größe besitzen, um den Durchgang von Wasserdampf durch diese hindurch zu erlauben.

2. Eine Elektrode gemäß Anspruch 1, bei welcher das poröse Material eine Mikrostruktur aus Knoten aufweist, die untereinander in der Weise durch Fibrillen verbunden sind, daß Porenzwischenräume zwischen den Fibrillen vorhanden sind, und die untereinander durch die Fibrillen verbundenen Knoten im wesentlichen miteinander verbunden sind.

3. Eine Elektrode gemäß Anspruch 2, bei welcher das poröse Material durch einen Prozeß der Fibrillisierung porös gemacht worden ist.

4. Eine Elektrode gemäß einem der Ansprüche 1 bis 3, bei welcher das leitfähige Pulver Ruß ist.

5. Eine Elektrode gemäß einem der Ansprüche 1 bis 3, bei welcher das leitfähige Pulver metallisch ist.

6. Eine Elektrode gemäß einem der vorhergehenden Ansprüche, bei welcher die Teilchengröße des leitfähigen Pulvers nicht mehr als 10 Mikron beträgt.

7. Eine Elektrode gemäß einem der vorhergehenden Ansprüche, die wenigstens eine Oberfläche aufweist, die zumindest teilweise mit einem Klebstoff beschichtet ist.

8. Eine Elektrode gemäß einem der vorhergehenden Ansprüche, bei welcher die Elektrode rechteckförmig ist.

9. Eine Elektrode gemäß einem der Ansprüche 1 bis 7, bei welcher die Elektrode von einer Kreisform oder Ellipsenform ist.

10. Eine Elektrode gemäß Anspruch 8 oder gemäß Anspruch 9, mit einem Verbindungsteil, das von einer Kante aus vorspringt.

## Revendications

1. Électrode, destinée notamment à être utilisée sur un tissu vivant, pour établir un contact électriquement conducteur sur une surface, ladite électrode possédant une partie destinée à venir en contact avec ladite surface, la partie de contact comprenant un matériau poreux, caractérisée en ce que ledit matériau poreux contient 5 à 90 % en poids de polytétrafluoroéthylène et 10 à 95 % en poids d'une poudre conductrice et en ce que les pores du matériau possèdent une taille suffisante pour permettre le passage de la vapeur d'eau.

2. Électrode selon la revendication 1, dans laquelle le matériau poreux possède une microstructure formée de noeuds interconnectés par des fibrilles, de sorte que des espaces vides existent entre les fibrilles, et les noeuds interconnectés par les fibrilles sont essentiellement connectés entre eux.

3. Électrode selon la revendication 2, dans laquelle le matériau poreux a été rendu poreux par un processus de fibrillation.

4. Électrode selon l'une quelconque des revendications 1 à 3, dans laquelle la poudre conductrice est du noir de carbone.

5. Électrode selon l'une quelconque des revendications 1 à 3, dans laquelle la poudre conductrice est métallique.

6. Électrode selon l'une quelconque des revendications précédentes, dans laquelle la taille des particules de la poudre conductrice n'est pas supérieure à 10 microns.

7. Électrode selon l'une quelconque des revendications précédentes, possédant au moins une face qui est au moins partiellement recouverte par un adhésif.

8. Électrode selon l'une quelconque des revendications précédentes, dans laquelle l'électrode a une forme rectangulaire.

9. Électrode selon l'une quelconque des revendications 1 à 7, dans laquelle l'électrode possède une forme circulaire ou elliptique.

10. Électrode selon la revendication 8 ou la revendication 9, possédant une partie de retournement qui fait saillie à partir d'un bord.
